# EUROPÄISCHE PATENTSCHRIFT

(11) **EP 1 242 628 B1**
(45) Veröffentlichungstag und Bekanntmachung des Hinweises auf die Patenterteilung: **13.09.2006**
(21) Anmeldenummer: 00990568.8
(22) Anmeldetag: 19.12.2000
(51) Int. Cl.: C12Q 1/68

(54) **VERFAHREN ZUR ANALYSE VON NUKLEINSÄURESEQUENZEN**
METHOD FOR ANALYZING NUCLEIC ACID SEQUENCES
PROCEDE D'ANALYSE DE SEQUENCES D'ACIDE NUCLEIQUE

(30) Priorität: 20.12.1999 DE 19963536
(43) Veröffentlichungstag der Anmeldung: 25.09.2002
(73) Patentinhaber: Epigenomics AG, 10178 Berlin (DE)
(72) Erfinder: GUT, Ivo, Glynne, F-75014 Paris (FR); BERLIN, Kurt, 14532 Stahnsdorf (DE)
(74) Vertreter: Schubert, Klemens
(86) Internationale Anmeldenummer: PCT/DE2000/004585
(87) Internationale Veröffentlichungsnummer: WO 2001/046460

(56) Entgegenhaltungen:
- WO-A-96/32504
- WO-A-97/37041
- WO-A-98/31830
- WO-A-99/29897
- JIANG-BAUCOM P AND GIRARD J E: "DNA typing of human leukocyte antigen sequence polymorphisms by peptide nucleic acid probes and MALDI-TOF mass spectrometry" ANALYTICAL CHEMISTRY, Bd. 69, 1997, Seiten 4894-4898, XP002174822
- ROSS ET AL: "DISCRIMINATION OF SINGLE-NUCLEOTIDE POLYMORPHISMS IN HUMAN DNA USING PEPTIDE NUCLEIC ACID PROBES DETECTED BY MALDI-TOF MASS SPECTROMETRY" ANALYTICAL CHEMISTRY, AMERICAN CHEMICAL SOCIETY. COLUMBUS, US, Bd. 69, Nr. 20, 15. Oktober 1997 (1997-10-15), Seiten 4197-4202, XP002113518 ISSN: 0003-2700 in der Anmeldung erwähnt
- OLEK A ET AL: "A modified an improved method for bisulphite based cytosine methylation analysis" NUCLEIC ACIDS RESEARCH, OXFORD UNIVERSITY PRESS, SURREY, GB, Bd. 24, Nr. 24, 1996, Seiten 5064-5066, XP002106408 ISSN: 0305-1048
- OLEJNIK J ET AL.: "Photorelease and MALDI analysis of oligonicleotides bound to solid surfaces through photocleavable biotin" BIOPHYSICAL JOURNAL, Bd. 74, Nr. 2Pt2, Februar 1998 (1998-02), Seite A296 XP001015375
- BERLIN K ET AL: "ANALYSIS OF NEGATIVELY 'CHARGE TAGGED' DNA BY MATRIX-ASSISTED LASERDESORPTION/IONIZATION TIME-OF-FLIGHT MASS SPECTROMETRY" PROCEEDINGS OF THE ANNUAL FREQUENCY CONTROL SYMPOSIUM. PHILADELPHIA, MAY 1987, NEW YORK, IEEE, US, Bd. SYMP. 41, 1987, Seiten 1739-1743, XP000971118
- GUT I G ET AL: "DNA AND MATRIX ASSISTED LASER DESORPTION IONIZATION MASS SPECTROMETRY" MOLECULAR BIOLOGY: CURRENT INNOVATIONS AND FUTURE TRENDS, HORIZON SCIENTIFIC PRESS, WYMONDHAM, GB, 1995, Seiten 147-157, XP000573516 in der Anmeldung erwähnt
- CHEN J ET AL: "A MICROSPHERE-BASED ASSAY FOR MULTIPLEXED SINGLE NUCLEOTIDE POLYMORPHISM ANALYSIS USING SINGLE BASE CHAIN EXTENSION" GENOME RESEARCH, COLD SPRING HARBOR LABORATORY PRESS, US, Bd. 10, Nr. 4, April 2000 (2000-04), Seiten 549-557, XP000927257 ISSN: 1088-9051 in der Anmeldung erwähnt

## Beschreibung

Die Erfindung betrifft ein Verfahren zur Analyse von Nukleinsäuresequenzen. Das Gebiet der Erfindung ist die Analyse von DNA oder RNA und insbesondere die Kopplung eines hochparallelisierbaren Probenaufbereitungsverfahrens mit einem Analyseverfahren mit hohem Durchsatz.

Unbekannte DNA kann charakterisiert werden, indem sie sequenziert wird. Dies ist die präziseste Art DNA zu analysieren, jedoch ist die Sequenzierung auch sehr aufwendig. Nur sehr kurze DNA-Ausschnitte (<1000 Nukleobasen) können auf einmal sequenziert werden. Wenn DNA Fragmente, die größer als diese 1000 Nukleobasen lang sind, in größerem Umfang analysiert werden sollen, ist es notwendig die DNA zu unterteilen, was die Methode verteuert. Ein praktikableres Verfahren ist es, partielle Information über einen Array von verschiedenen Ziel-DNAs zu suchen. Ein Array mit vielen tausend Ziel-DNAs kann auf einer Festphase immobilisiert und anschließend alle Ziel-DNAs gemeinsam auf das Vorhandensein einer Sequenz mittels einer Sonde (Nukleinsäure mit komplementärer Sequenz) untersucht werden (Scholler, P., Karger, A.E., Meier-Ewert, S., Lehrach, H., Delius, H. and Hoheisel, J.D. 1995. Fine-mapping of shotgun template-libraries; an efficient strategy for the systematic sequencing of genomic DNA. Nucleic Acids Res. 23: 3842-3849). Eine Übereinstimmung in der Ziel-DNA mit der Sonde kommt durch eine Hybridisation der zwei Teile miteinander zustande. Sonden können beliebige Nukleinsäuresequenzen von beliebiger Länge sein. Es existieren verschiedene Verfahren für die Auswahl von optimalen Bibliotheken von Sondensequenzen, welche minimal miteinander überlappen. Sondensequenzen können auch gezielt zusammengestellt werden, um bestimmte Ziel-DNA Sequenzen aufzufinden. Oligofingerprinting ist ein Ansatz, bei welchem diese Technologie zum Einsatz kommt. Eine Bibliothek von Ziel-DNAs wird mit kurzen Nukleinsäuresonden abgetastet. Meist sind hier die Sonden nur 8-12 Basen lang. Es wird jeweils eine Sonde auf einmal an eine auf einer Nylonmembran immobilisierte Ziel-DNA-Bibliothek hybridisiert. Die Sonde ist radioaktiv markiert und die Hybridisierung wird anhand der Lokalisierung der Radioaktivität beurteilt. Für die Abtastung eines immobilisierten DNA-Arrays sind auch schon fluoreszent markierte Sonden verwendet worden. (Guo, Z., Guilfoyle, R.A., Thiel, A.J., Wang, R. and Smith, L.M. 1994. Direct fluorescence analysis of genetic polymorphisms by hybridization with oligonucleotides arrays of glass supports. Nucleic Acids Res. 22: 5456-5465).

Als Sonden kommen jegliche Moleküle in Frage, die sequenzspezifisch mit einer Ziel-DNA wechselwirken können. Am gängigsten sind Oligodeoxyribonukleotide. Jedoch bietet sich jede Modifikation von Nukleinsäuren an, z.B. Peptide Nucleic Acids (PNA), (Nielsen, P.E., Buchardt, O., Egholm, M. and Berg, R.H. 1993. Peptide nucleic acids. US Patent. 5,539,082; Buchardt, O., Egholm, M., Berg, R.H. and Nielsen, P.E. 1993. Peptide nucleic acids and their potential applications in biotechnology. Trends in Biotechnology, 11: 384-386), Phosphorothioatoligonukleotide oder Methylphosphonatoligonukleotide. Die Spezifität einer Sonde ist sehr wesentlich. Peptide Nucleic Acids haben ein ungeladenes Rückgrat, welches gleichzeitig chemisch sehr stark von der gängigen Zucker-Phosphat Struktur des Rückgrats in Nukleinsäuren abweicht. Das Rückgrat einer PNA hat eine Amidsequenz anstelle des Zucker-Phosphat Rückgrats gewöhnlicher DNA. PNA hybridisiert sehr gut mit DNA komplementärer Sequenz. Die Schmelztemperatur eines PNA/DNA-Hybrids ist höher als die des entsprechenden DNA/DNA-Hybrids und die Abhängigkeit der Hybridisierung von Puffersalzen ist relativ gering.

Matrix-assistierte Laser Desorptions/Ionisations Massenspektrometrie (MALDI) ist eine sehr leistungsfähige Entwicklung für die Analyse von Biomolekülen (Karas, M. and Hillenkamp, F. 1988. Laser desorption ioniztion of proteins with molecular masses exceeding 10000 daltons. Anal. Chem. 60: 2299-2301). Ein Analytmolekül wird in eine lichtabsorbierende Matrix eingebettet. Durch einen kurzen Laserpuls wird die Matrix verdampft und das Analytmolekül so unfragmentiert in die Gasphase befördert. Durch Stöße mit Matrixmolekülen wird die Ionisation des Analyts erreicht. Eine angelegte Spannung beschleunigt die Ionen in ein feldfreies Flugrohr. Auf Grund ihrer verschiedenen Massen werden Ionen unterschiedlich stark beschleunigt. Kleinere Ionen erreichen den Detektor früher als größere.

MALDI eignet sich ausgezeichnet zur Analyse von Peptiden und Proteinen. Die Analyse von Nukleinsäuren ist etwas schwieriger (Gut, I.G. and Beck, S. 1995. DNA and Matrix Assisted Laser Desorption Ionization Mass Spectrometry. Molecular Bioogy: Current Innovations and Future Trends. 1: 147-157.) Für Nukleinsäuren ist die Empfindlichkeit etwa 100-mal schlechter als für Peptide und nimmt mit zunehmender Fragmentgröße überproportional ab. Für Nukleinsäuren, die ein vielfach negativ geladenes Rückgrat haben, ist der Ionisationsprozeß durch die Matrix wesentlich ineffizienter. Für MALDI spielt die Wahl der Matrix eine eminent wichtige Rolle. Für die Desorption von Peptiden sind einige sehr leistungsfähige Matrices gefunden worden, die eine sehr feine Kristallisation ergeben. Für DNA sind zwar mittlerweile einige ansprechende Matrices gefunden worden, jedoch wurde dadurch der Empfindlichkeitsunterschied nicht verringert. Der Empfindlichkeitsunterschied kann verringert werden, indem die DNA chemisch so modifiziert wird, daß sie einem Peptid ähnlicher wird. Phosphorothioatnukleinsäuren, bei denen die gewöhnlichen Phosphate des Rückgrats durch Thiophosphate substituiert sind, lassen sich durch einfache Alkylierungschemie in eine ladungsneutrale DNA umwandeln (Gut, I.G. and Beck, S. 1995. A procedure for selective DNA alkylation and detection by mass spectrometry. Nucleic Acids Res. 23: 1367-1373). Die Kopplung eines "charge tags" an diese modifizierte DNA resultiert in der Steigerung der Empfindlichkeit in den gleichen Bereich wie er für Peptide gefunden wird. Ein weiterer Vorteil von "charge tagging" ist die erhöhte Stabilität der Analyse gegen Verunreinigungen, die den Nachweis unmodifizierter Substrate stark erschweren. PNAs und Methylphosphonatoligonukleotide sind mit MALDI untersucht worden und lassen sich so analysieren.Butler, J.M., Jiang-Baucom, P., Huang, M., Belgrader, P. and Girard, J. 1996. Peptide nucleic acid characterization by MALDI-TOF mass spectrometry. Anal. Chem. 68: 3283-3287; Keough, T., Baker, T.R., Dobson, R.L.M., Lacey, M.P., Riley, T.A., Hasselfield, J.A. and Hesselberth, P.E. 1993. Antisense DNA oligonucleotides II: the use of matrix-assisted laser desorption/ionization mass spectrometry for the sequence verification of methylphosphonate oligodeoxyribonucleotides. Rapid Commun. Mass Spectrom. 7: 195-200; Ross, P.L., Lee, K. and Belgrader, P. 1997. Discrimination of singlenucleotide polymorphisms in human DNA using peptide nucleic acid probes detected by MALDI-TOF mass spectrometry. Anal. Chem. 69: 4197-4202).

Kombinatorische Synthesen (Lowe, G. 1995. Combinatorial Chemistry. Chem. Soc. Rev. 24: 309), d.h. die Herstellung von Substanzbibliotheken ausgehend von einem Gemisch von Vorstufen, werden sowohl auf fester als auch in flüssiger Phase durchgeführt. Vor allem die kombinatorische Festphasensynthese hat sich frühzeitig etabliert, da in diesem Fall die Abtrennung von Nebenprodukten besonders einfach ist. Nur die an den Support gebundenen Zielverbindungen werden in einem Waschschritt zurückbehalten und am Ende der Synthese durch das gezielte Spalten eines Linkers isoliert. Diese Technik erlaubt auf einfachem Wege die gleichzeitige Synthese einer Vielzahl verschiedener Verbindungen an einer Festphase und somit den Erhalt von chemisch "reinen" Substanzbibliotheken. Daher sind die Verbindungsklassen, die auch in nicht kombinatorischen, konventionellen Synthesen auf einer Festphase synthetisiert werden, der kombinatorischen Chemie besonders leicht zugänglich und werden demzufolge auch breit verwendet. Dies trifft vor allem auf Peptid-, Nukleinsäure- und PNA-Bibliotheken zu.

Die Synthese von Peptiden erfolgt durch Binden der ersten N-geschützten Aminosäure (z.B. Boc) an den Support, nachfolgende Entschützung und Reaktion der zweiten Aminosäure mit der freigewordenen NH₂-Gruppe der ersten. Nicht reagierte Aminofunktionen werden in einem weiteren "Capping" Schritt einer Weiterreaktion im nächsten Synthesezyklus entzogen. Die Schutzgruppe an der Aminofunktion der zweiten Aminosäure wird entfernt und der nächste Baustein kann gekoppelt werden. Zur Synthese von Peptidbibliotheken wird ein Gemisch von Aminosäuren in einem oder mehreren Schritten verwendet. Die Synthese von PNA und PNA-Bibliotheken erfolgt sinngemäß. Nukleinsäure-Bibliotheken werden meist durch Festphasensynthese mit Gemischen verschiedener Phosphoramidit-Nucleoside erhalten. Dies kann auf kommerziell erhältlichen DNA-Synthesizern ohne Veränderungen in den Syntheseprotokollen durchgeführt werden.

Verschiedene Arbeiten zur kombinatorischen Synthese von PNA Bibliotheken sind publiziert worden. Diese Arbeiten behandeln den Aufbau von kombinatorischen Sequenzen, d.h. die Synthese von PNAs in denen einzelne, spezifische Basen in der Sequenz durch degenerierte Basen ersetzt werden und dadurch zufällige Sequenzvarianz erreicht wird. Die Verwendung massenspektrometrischer Methoden für die Analyse kombinatorischer Bibliotheken ist mehrfach beschrieben worden (z. B. Carr, S.A., Benkovic, S.J., Winograd, N. 1996. Evaluation of Mass Spectrometric Methods Applicable to the Direct Analysis of Non-Peptide Bead-Bound Combinatorial Libraries. Anal. Chem. 68: 237).

Es existieren verschiedene Verfahren um DNA zu immobilisieren. Das bekannteste Verfahren ist die Festbindung einer DNA, welche mit Biotin funktionalisiert ist, an eine Streptavidin-beschichtete Oberfläche (Uhlen, M. et al. 1988, Nucleic Acids Res. 16, 3025-3038). Die Bindungsstärke dieses Systems entspricht einer kovalenten chemischen Bindung ohne eine zu sein. Um eine Ziel-DNA kovalent an eine chemisch vorbereitete Oberfläche binden zu können, bedarf es einer entsprechenden Funktionalität der Ziel-DNA. DNA selbst besitzt keine Funktionalisierung, die geeignet ist. Es gibt verschiedene Varianten in eine Ziel-DNA eine geeignete Funktionalisierung einzuführen: Zwei leicht zu handhabende Funktionalisierungen sind primäre, aliphatische Amine und Thiole. Solche Amine werden quantitativ mit N-Hydroxy-succinimidestern umgesetzt und Thiole reagieren unter geeigneten Bedingungen quantitativ mit Alkyliodiden. Eine Schwierigkeit besteht im Einführen einer solchen Funktionalisierung in eine DNA. Die einfachste Variante ist die Einführung durch einen Primer einer PCR. Gezeigte Varianten benützen 5'-modifizierte Primer (NH₂ und SH) und einen bifunktionalen Linker.

Ein wesentlicher Bestandteil der Immobilisierung auf einer Oberfläche ist ihre Beschaffenheit. Bis jetzt beschriebene Systeme sind hauptsächlich aus Silizium oder Metall (magnetic beads). Eine weitere Methode zur Bindung einer Ziel-DNA basiert darauf, eine kurze Erkennungssequenz (z.B. 20 Basen) in der Ziel-DNA zur Hybridisierung an ein oberflächenimmobilisiertes Oligonukleotid zu verwenden. Es sind auch enzymatische Varianten zur Einführung von chemisch aktivierten Positionen in eine Ziel-DNA beschrieben worden. Hier wird an einer Ziel-DNA enzymatisch eine 5'-NH₂-Funktionalisierung durchgeführt.

Für die Abtastung eines immobilisierten DNA-Arrays sind vielfach fluoreszent markierte Sonden verwendet worden. Besonders geeignet sind für die Fluoreszenzmarkierung ist das einfache Anbringen von Cy3 und Cy5 Farbstoffen am 5'OH der jeweiligen Sonde. Die Detektion der Fluoreszenz der hybridisierten Sonden erfolgt beispielsweise über ein Konfokalmikroskop. Die Farbstoffe Cy3 und Cy5 sind, neben vielen anderen, kommerziell erhältlich.

Auch Methoden zur massenspektrometrischen Analyse von DNA-Arrays sind beschrieben. So ist in der Patentanmeldung WO 99/29897 (Anmelder: Max-Planck-Gesellschaft) ein Verfahren offenbart, bei dem die zu untersuchenden Nukleinsäuremoleküle auf einen Träger immobilisiert werden. Danach wird ein Satz von Sonden unterschiedlicher Sequenz und unterschiedlicher Masse an die zu untersuchende DNA hybridisiert. Schließlich werden die Sonden mittels Elektrospray Massenspektrometrie analysiert.

Eine massenspektrometrische Methode zur Analyse von Cytosin-Methylierungen ist in der Patenanmeldung WO 00/44934 (Anmelder Epigenomics) beschrieben. Dabei wird die zu untersuchende DNA zunächst chemisch umgewandelt, anschließend amplifiziert und an eine Oberfläche gebunden. Danach erfolgt eine Hybridisierung mit spezifischen, massesensitiven Sonden und zuletzt eine massenspektrometrische Analyse der Sonden.

Eine Übersicht über den Stand der Technik in der Oligomer Array Herstellung läßt sich auch einer im Januar 1999 erschienen Sonderausgabe von Nature Genetics (Nature Genetics Supplement, Volume 21, January 1999) und der dort zitierten Literatur entnehmen.

Eine relativ neue und die mittlerweile am häufigsten angewandte Methode zur Untersuchung von DNA auf 5-Methylcytosin beruht auf der spezifischen Reaktion von Bisulphit mit Cytosin, das nach anschließender alkalischer Hydrolyse in Uracil umgewandelt wird, welches in seinem Basen-Paarungsverhalten dem Thymidin entspricht. 5-Methylcytosin wird dagegen unter diesen Bedingungen nicht modifiziert. Damit wird die ursprüngliche DNA so umgewandelt, daß Methylcytosin, welches ursprünglich durch sein Hybridisierungsverhalten vom Cytosin nicht unterschieden werden kann, jetzt durch "normale" molekularbiologische Techniken als einzig verbliebenes Cytosin beispielsweise durch Amplifikation und Hybridisierung oder Sequenzierung nachgewiesen werden kann. Alle diese

Techniken beruhen auf Basenpaarung, welche jetzt voll ausgenutzt werden kann. Der Stand der Technik, was die Empfindlichkeit betrifft, wird durch ein Verfahren definiert, welches die zu untersuchende DNA in einer Agarose-Matrix einschließt, dadurch die Diffusion und Renaturierung der DNA (Bisulphit reagiert nur an einzelsträngiger DNA) verhindert und alle Fällungs- und Reinigungsschritte durch schnelle Dialyse ersetzt (Olek, A. et al., Nucl. Acids. Res. 1996, 24, 5064-5066). Mit dieser Methode können einzelne Zellen untersucht werden, was das Potential der Methode veranschaulicht. Allerdings werden bisher nur einzelne Regionen bis etwa 3000 Basenpaare Länge untersucht, eine globale Untersuchung von Zellen auf Tausende von möglichen Methylierungsereignissen ist nicht möglich. Allerdings kann auch dieses Verfahren keine sehr kleinen Fragmente aus geringen Probenmengen zuverlässig analysieren. Diese gehen trotz Diffusionsschutz durch die Matrix verloren.

Eine Übersicht über die weiteren bekannte Möglichkeiten, 5-Methylcytosine nachzuweisen, kann auch dem folgenden Übersichtsartikel entnommen werden: Rein, T., DePamphilis, M. L., Zorbas, H., Nucleic Acids Res. 1998, 26, 2255.

Die Bisulphit-Technik wird bisher bis auf wenige Ausnahmen (z.B. Zeschnigk, M. et al., Eur. J. Hum. Gen. 1997, 5, 94-98) nur in der Forschung angewendet. Immer aber werden kurze, spezifische Stücke eines bekannten Gens nach einer Bisulphit-Behandlung amplifiziert und entweder komplett sequenziert (Olek, A. and Walter, J., Nat. Genet. 1997, 17, 275-276) oder einzelne Cytosin-Positionen durch eine "Primer-Extension-Reaktion" (Gonzalgo, M. L. and Jones, P.A., Nucl. Acids. Res. 1997, 25, 2529-2531) oder Enzymschnitt (Xiong, Z. and Laird, P.W., Nucl. Acids. Res. 1997, 25, 2532-2534) nachgewiesen. Zudem ist auch der Nachweis durch Hybridisierung beschrieben worden (Olek et al., WO99 28498).

Weitere Publikationen, die sich mit der Anwendung der Bisulfit-Technik zum Methylierungsnachweis bei einzelnen Genen befassen, sind: Xiong, Z. and Laird, P.W. (1997), Nucl. Acids Res. 25, 2532; Gonzalgo, M.L. and Jones, P.A. (1997), Nucl. Acids Res. 25, 2529; Grigg, S. and Clark, S. (1994), Bioessays 16, 431; Zeschnik, M. et al. (1997), Human Molecular Genetics 6, 387; Teil, R. et al. (1994), Nucl. Acids Res. 22, 695; Martin, V. et al. (1995), Gene 157, 261; WO 97/46705, WO 95/15373 und WO 97/45560.

Kodierte Partikel (Beads) finden schon seit einiger Zeit in sehr unterschiedlichen Gebieten ihre Anwendung. Farbkodierte Beads sind zur parallelen Diagnostik von T- und B-Zellen eingesetzt worden (Baran und Parker, Am. J. Clin. Pathol. 1985, 83, 182-9). Mit radiaoaktivem Indium versetzte Beads sind als Indikatoren der Motilität des gastrointestalen Trakts verwendet worden (Dormehl et al Eur. J. Nucl. Med. 1985, 10, 283-5). Kürzlich sind zwei Firmen gegründet worden, die mit farbkodierten Kunststoffkügelchen hoch-parallele Diagnostik betreiben wollen (Luminex www.luminexcorp.com und Illumina www.illumina.com). Diese Firmen verwenden 100 verschieden farbmarkierte Beads, an denen entsprechend viele verschiedene Sonden angebracht werden können. Dadurch können in einer Reaktion 100 verschiedene Parameter abgefragt werden, was z.B. 100 verschiedene diagnostische Tests sein könnten (Chen J, Iannone MA, Li M-S, Taylor D, Rivers P, Nelsen AJ, Slentz-Kesler KA, Roses A, Weiner MP, "A microsphere-based assay for multiplexed single nucleotide polymorphism analysis using single base chain extension", Genome Research 10:549-557; Iannone MA, Taylor JD, Chen J, Li M-S, Rivers P, Slentz KA, Weiner MP, "Multiplexed single nucleotide polymorphism genotyping by oligonucleotide ligation and flow cytometry", Cytometry 39:131-140; Healey BG, Matson RS, Walt DR, "Fiberoptic DNA sensor array capable of detecting point mutations", Analytical Chemistry 251:270-279).

Ein Übersichtsartikel zur Verwendung codierter Beads findet sich in der November/Dezember-Ausgabe 1998 von Modern Drug Discovery (Czarnik A.W.: Illuminating the SNP genome code. Vol 1, No.2, 49-50). Beschrieben ist dort u.a. ein Verfahren zur Analyse von Nukleinsäuren, wobei mit einem Fluoreszenzfarbstoff codierte Beads verwendet werden. Die Beads tragen zudem eine Oligonukleotidsonde. Die zu untersuchende DNA wird fluoreszenzmarkiert und danach an die Sonden auf den Beads hybridisiert. Anschließend werden die Fluoreszenzfarbstoffe detektiert.

Aufgabe der vorliegenden Erfindung ist es ein Analyseverfahren zu schaffen, welches sich durch die Kopplung eines hoch parallelisierbaren Probenaufbereitungsverfahrens mit einem Analyseverfahren mit hohem Durchsatz auszeichnet.

Die Aufgabe wird dadurch gelöst, daß ein in Anspruch 1 beschriebenes Verfahren zur Analyse von Nukleinsäuresequenzen geschaffen wird,

Dabei ist es erfindungsgemäß bevorzugt, daß die in Schritt a) hybridisierten Nukleinsäurefragmente DNA sind oder daß die in Schritt a) hybridisierten Nukleinsäurefragmente RNA sind oder daß die in Schritt a) hybridisierten Nukleinsäurefragmente durch die Polymerase Kettenreaktion erhältlich sind oder daß die in Schritt a) hybridisierten Nukleinsäurefragmente durch Restriktionsverdau erhältlich sind oder daß die in Schritt a) hybridisierten Nukleinsäurefragmente durch Behandlung mit einer reversen Transkriptase und anschließender Polymerase Kettenreaktion erhältlich sind.

Erfindungsgemäß bevorzugt ist ferner, daß die in Schritt b) verwendeten Sonden selbst Nukleinsäuren sind.

Weiterhin ist erfindungsgemäß bevorzugt, daß die in Schritt b) verwendeten Sonden PNA, Alkylphosphonat-DNA, Phosphorothioat-DNA oder alkylierte Phosphorothioat-DNA sind.

Erfindungsgemäß bevorzugt ist außerdem, daß die in Schritt b) verwendeten Sonden entweder eine einzelne positive oder negative Nettoladung tragen oder daß die in Schritt b) verwendeten Sonden chemische Gruppen tragen, welche deren Molekülmasse verändern oder daß die in Schritt b) verwendeten Sonden abspaltbare Gruppen enthalten, welche über deren Masse identifizierbar sind.

Erfindungsgemäß bevorzugt ist auch, daß jede der in Schritt b) verwendeten Sondensequenzen über deren Sondenmasse identifizierbar ist. Bevorzugt ist ferner, daß die in Schritt b) verwendeten Sonden durch kombinatorische Synthese erhältlich sind.

Beim erfindungsgemäßen Verfahren ist auch bevorzugt, daß die in Schritt a) verwendeten Träger über Fluoreszenzfarbstoffe codiert sind oder daß die in Schritt a) verwendeten Träger über absorbierende Farbstoffe codiert sind oder daß die in Schritt a) verwendeten Träger über Chemiluminiszenz codiert sind oder daß die in Schritt a) verwendeten Träger über Transponder codiert sind.

Bevorzugt ist ferner, daß die in Schritt a) verwendeten Träger über Nuklide codiert sind, welche mittels Elektronenspinresonanz, Kernspinresonanz oder radioaktiven Zerfall nachweisbar sind oder daß die in Schritt a) verwendeten Träger über chemische Markierungen codiert sind, welche massenspektrometrisch nachweisbar sind.

Ferner ist erfindungsgemäß bevorzugt, daß man je Träger nur eine definierte Sequenz bindet. Wahlweise ist auch bevorzugt, daß man je Träger mehrere definierte Sequenzen bindet.

Bevorzugt ist aber auch, daß man an den Trägern zu den Primern aus der Amplifikation komplementäre Sequenzen bindet.

Bei einer erfindungsgemäßen Variante ist ferner bevorzugt, daß man die Schritte a) und b) gleichzeitig ausführt.

Außerdem ist bevorzugt, daß die in der Amplifikation verwendeten Primer Fluoreszenzmarkierungen tragen, die eine Vorauswahl der Träger vor der Analyse zulassen.

Besonders bevorzugt ist die erfindungsgemäße Variante des erfindungsgemäßen Verfahren, wobei man die Träger vor der Durchführung des Schrittes c) aufreiht, identifiziert und nacheinander einer Analyse zuführt.

Gleichfalls bevorzugt ist es, daß man die Träger vor der Durchführung des Schrittes c) derart auf eine Oberfläche verteilt, daß an vorbestimmten Orten jeweils nur ein Träger positioniert ist.

Das erfindungsgemäße Verfahren bevorzugt ferner, daß man die Sonden vor, bei oder nach der Einführung ins Massenspektrometer vom Träger löst.

Außerdem ist bevorzugt, daß man zur Desorption eine Matrix zugibt.

Besonders bevorzugt ist es, daß man die Analyse mittels MALDI-Massenspektrometrie durchführt.

In einer anderen Variante des erfindungsgemäßen Verfahrens ist es bevorzugt, daß man die Analyse mittels ESI-Massenspektrometrie durchführt.

Bevorzugt ist es auch, daß man eine Ionenfalle in der massenspekrometrischen Analyse einsetzt.

Eine besonders bevorzugte Variante des Verfahrens ist es, daß die Identifikation des Trägers und die Analyse der hybridisierten Sonden in einem Verfahrensschritt erfolgen.

Ganz besonders ist es erfindungsgemäß bevorzugt, daß man die in Schritt a) eingesetzte DNA zuvor mit Sulfit oder Disulfit oder einer anderen Chemikalie derart behandelt, daß alle nicht an der 5-Position der Base methylierten Cytosinbasen derart verändert werden, daß eine dem Basenpaarungsverhalten nach unterschiedliche Base entsteht, während die an der 5-Position methylierten Cytosine unverändert bleiben.

Ein weiterer Gegenstand der vorliegenden Erfindung ist ein Kit gemäß Anspruch 26,

Beschrieben wird also ein Verfahren zur Analyse von Nukleinsäuresequenzen, das sich durch die Ausführung folgender Schritte kennzeichnet:
Im ersten Schritt werden beliebige Nukleinsäurefragmente an komplementäre Sequenzen, welche auf codierten Trägern immobilisiert sind, hybridisiert.
Die Nukleinsäurefragmente können dabei DNA und/oder RNA sein.
In einer besonders bevorzugten Variante des Verfahrens werden die hybridisierten DNA-Fragmente zuvor durch die Polymerase Kettenreaktion hergestellt. In einer besonders bevorzugten Variante des Verfahrens geht der Polymerase Kettenreaktion eine Behandlung von RNA mit einer reversen Transkriptase voraus.
In einer weiteren bevorzugten Variante des Verfahrens werden die hybridisierten Nukleinsäurefragmente durch Restriktionsverdau hergestellt.

Die Codierung der Träger erfolgt bevorzugt über Fluoreszenzfarbstoffe und/oder über absorbierende Farbstoffe und/oder über Chemiluminiszenz und/oder über Transponder und/oder über Elektronenspinresonanz und/oder über Kernspinresonanz und/oder radiaktiven Zerfall.

In einer besonders bevorzugten Variante des Verfahrens erfolgt die Codierung der Träger über chemische Markierungen, welche sich massenspektrometrisch nachweisen lassen.

An jeden Träger können spezifisch jeweils eine definierte Ziel-Sequenz oder auch mehrere unterschiedliche, definierte Ziel-Sequenzen gebunden werden. In einer besonders bevorzugten Variante des Verfahrens sind an den Trägern zu den Primern aus der Amplifikation komplementäre Sequenzen gebunden.

In einer besonders bevorzugten Variante des Verfahrens wird die eingesetzte DNA zuvor bevorzugt mit Sulfit oder Disulfit oder aber einer anderen Chemikalie derart behandelt, daß alle nicht an der 5-Position der Base methylierten Cytosinbasen derart verändert werden, daß eine dem Basenpaarungsverhalten nach unterschiedliche Base entsteht, während die an der 5-Position methylierten Cytosine unverändert bleiben. Diese Ausführung kann zur Identifikation von Cytosin-Methylierungsmustern in DNA-Proben verwendet werden.

Im zweiten Verfahrensschritt wird eine Hybridisierung von Sonden an die im ersten Schritt hybridisierten Nukleinsäurefragmente durchgeführt.

In einer bevorzugten Ausführungsform des Verfahrens sind die verwendeten Sonden selbst DNA. In einer besonders bevorzugten Variante sind die Sonden PNA (Peptide Nucleic Acids) und/oder Alkylphosphonat-Oligonukleotide und/oder Phosphorothioat-DNA oder alkylierte Phosphorothioat-DNA oder Chimäre dieser Verbindungsklassen.

In einer weiteren, besonders bevorzugten Variante des Verfahrens tragen die verwendeten Sonden entweder eine positive oder negative einzelne Nettoladung. In einer weiteren bevorzugten Variante tragen die Sonden chemische Gruppen, die der Veränderung ihrer Molekülmasse dienen.

In einer weiteren bevorzugten Variante enthalten die verwendeten Sonden abspaltbare Gruppen, deren Masse wiederum zu ihrer Identifizierung genutzt werden kann.

In einer besonders bevorzugten Ausführung des Verfahrens ist die Zusammensetzung einer Sondenbibliothek so gewählt, daß jede der verwendeten Sondensequenzen über die Sondenmasse eindeutig identifizierbar ist. In einer besonders bevorzugten Variante des Verfahrens werden die Sondenbibliotheken durch kombinatorische Synthese hergestellt.

In einer besonders bevorzugten Ausführung des Verfahrens werden der erste und zweite Schritt des Verfahrens gleichzeitig ausgeführt. In einer weiteren Variante wird der zweite Verfahrensschritt vor dem ersten durchgeführt.

Im dritten Schritt des Verfahrens wird eine sequentielle Identifikation der codierten Träger und Analyse der an diesen gebundenen Sonden in einem Massenspektrometer durchgeführt und in einem weiteren Schritt die erhaltene Masseninformation zu den Sequenzen der verwendeten Sonden zugeordnet. Zur Identifikation der Beads dient die oben erwähnte Codierung. Das Ablesen der Codierung kann vor, während oder nach der Detektion der hybridisierten Sonden erfolgen.

In einer besonders bevorzugten Verfahrensausführung tragen die in der Amplifikation verwendeten Primer Fluoreszenzmarkierungen, die eine Vorauswahl der Träger vor der Analyse zulassen.

In einer weiteren bevorzugten Variante des Verfahrens werden die Träger vor der Analyse aufgereiht und nacheinander einer Analyse zugeführt. Alternativ können die Träger vor der Analyse derart auf eine Oberfäche verteilt werden, daß an vorbestimmten Orten jeweils nur ein Träger positioniert wird.

In einer besonders bevorzugten Variante des Verfahrens werden die Sonden vor, bei oder nach der Einführung ins Massenspektrometer vom jeweiligen Träger gelöst.

In einer besonders bevorzugten Variante des Verfahrens wird die Analyse mittels MALDI-Massenspektrometrie durchgeführt. Bevorzugt wird zur besseren Desorption im Massenspektrometer eine Matrix zugegeben. Alternativ kann die Analyse mittels ESI-Massenspektrometrie durchgeführt werden. Bevorzugt ist ebenfalls die Verwendung einer Ionenfalle in der massenspektrometrischen Analyse.

In einer besonders bevorzugten Variante des Verfahrens erfolgt die Identifikation des Trägers und die Analyse der hybridisierten Sonden in einem Verfahrensschritt.

Im letzten Schritt erfolgt ein Abgleich der Informationen mit einer Datenbank. Zuvor werden die Analyseergebnisse mit der Codierung der Beads assoziiert. Somit ist bekannt, welche Sondenmuster zu welcher Ausgangssequenz auf den Beads korrelieren.

Weiterer Gegenstand der Erfindung ist ein Kit gemäß Anspruch 26.

Die folgenden Beispiele erläutern die Erfindung.

### Beispiel 1

Bindung von Oligonukleotiden an kodierte Partikel.

Die kodierten Partikel sind carboxylatbeschichtet. Die Carboxylatgruppen werden mit Acylisoharnstoff (1-Ethyl-3-(3-Dimethylaminopropyl) Carbodiimid Hydrochlorid) zur Aktivierung verestert. Anschliessend wird der Sulfo-NHS Ester gebildet. An diesen wird ein aminomodifiziertes Oligonukleotid gebunden. Das aminomodifizierte Oligonukleotid lässt sich auch unmittelbar an die mit 1-Ethyl-3-(3-Dimethylaminopropyl) Carbodiimid Hydrochlorid aktivierten kodierten Partikel binden.

### Beispiel 2

### Codierung der Beads mit Massenlabeln

Die Beads werden wie oben beschrieben aktiviert und nachfolgend wird ein photolabiler Linker, wie auch aus der Peptidsynthese bekannt, gekoppelt. Nachfolgend wird das Oligomer, welches die Proben-DNA binden soll, sowie die zur Codierung verwendeten Moleküle, in diesem Fall Tripeptide mit charakteristischer Masse, an die Linker gekoppelt. Dabei kommt bekannte Peptidchemie zum Einsatz, wie sie auch unter anderem in der PNA-Synthese verwendet wird (HATU als Aktivator, alternativ EDC).

### Beispiel 3

### Hybridisierung der Proben

Das PCR-Produkt wird an 30mer Oligonukleotide, die an das Bead immobilisiert sind, hybridisiert, unter Bedingungen wie sie dem Fachmann geläufig sind (T = 41°C, NH₄Cl 0,7 M, Citrat 0,07 M, -Larouylsarcosinat 3,6 %). Das PCR-Produkt wird auf an sich bekannte Art bevorzugt asymmetrisch hergestellt, in dem der Forward oder reverse-Primer in ca. 6fach höherer Konzentration eingesetzt wird. Nach der 1. Hybridisierung wird zunächst mit Puffer und dann sehr kurz mit aqua dest. nachgewaschen.

### Beispiel 4

### Hybridisierung der Sonden

Die Hybridisierung einer PNA-Sonde an die inzwischen an das Bead gebundene DNA-Probe erfolgt bei T = 32°C (11mer Sonde) in einem für diese Zwecke geeigneten Puffer, z.B. NH₄Cl 0,23 M, Citrat 0,023 M, Larouylsarcosinat 3,6 %. Nach der 2. Hybridisierung wird ebenfalls mit dem Hybridisierungspuffer und sehr kurz mit aqua dest. nachgewaschen.

### Beispiel 5

### Massenspektrometrische Identifikation der codierten Beads mit gleichzeitiger Analyse

### Variante 1:

Die massencodierten Beads mit den hybridisierten Sonden werden in eine Mikrotiterplatte verteilt, pro Well bevorzugt ein Bead. Die Mikrotiterplatte wird nachfolgend mit einem wässrigen Puffer befüllt, im einfachsten Fall wird destilliertes Wasser verwendet. Die Mikrotiterplatte wird derart belichtet, dass es zu einer Spaltung des photolabilen Linkers kommt, entsprechend den Spezifikationen des Herstellers des Linkers, beispielsweise mit einer Hg-Hochdrucklampe. Die Lösung wird entweder unmittelbar in einem ESI-Massenspektrometer vermessen oder aber nach Vermischen mit einer Matrix (s.u.) auf einem MALDI-Probenträger eingetrocknet und nachfolgend vermessen.

### Variante 2:

Die massencodierten Beads mit den hybridisierten Sonden werden zusammen mit einer Matrix direkt auf einen MALDI-Probenträger aufgebracht. Die Positionen der Beads auf dem Probenträger werden identifiziert und die hybridisierten Sonden sowie die Massencodierung in einem Schritt identifiziert. Dabei werden die photolabilen Linker durch das eingestrahlte Laserlicht gespalten und damit auch die Massencodierungen freigesetzt.

### Beispiel 6

### Analyse auf dem Massenspektrometer

Die Beads mit den an diese hybridisierten Sonden werden auf die Wells einer Mikrotiterplatte verteilt, wie auch für kombinatorische Festphasensynthesen üblich, wobei jedes Well bevorzugt nur ein Bead enthalten soll. Die Wells werden mit einem Puffer zur Aufnahme der Sonden gefüllt, im einfachsten Fall kann destilliertes Wasser verwendet werden. Werden PNAs als Sonden eingesetzt, so hat sich die Verwendung von 0,1% TFA bewährt.

Die hybridisierten Sonden werden entweder thermisch oder mittels eines denaturierenden Reagenzes, wie z.B. 40% Formamid, von den Beads abgelöst. Nun werden die Lösungen direkt auf den Probenträger des Massenspektrometers aufgebracht. In diesem Beispiel wird ein Bruker Biflex Massenspektrometer mit Scout 384 Ionenquelle verwendet. Dadurch ist es möglich, dass die Lösungen aus einer 384er Mikrotiterplatte direkt mittels Pins übertragen werden können, da der Abstand der Wells in der Mikrotiterplatte dem Abstand der Proben auf dem Probenträger entspricht. Nachfolgend wird gleichermaßen die MALDI Matrix aufgetragen, wobei je nach Sonde unterschiedliche Varianten zum Einsatz kommen können. Für PNA Sonden hat sich beispielsweise eine 1%ige Lösung von alpha-Cyano-4-hydroxyzimtsäuremethylester und alpha-Cyano-4-methoxyzimtsäure im Verhältnis 1:1 bewährt.

Die Massen der Sonden werden wie dem Fachmann bekannt bestimmt und aus dem Muster auf die Sequenzen der an die Beads gebundenen DNA Fragmente geschlossen.

## Patentansprüche

1. Verfahren zur Analyse von Nukleinsäuresequenzen, **dadurch gekennzeichnet, dass** man die folgenden Schritte ausführt:
a) Hybridisierung von Nukleinsäurefragmenten an komplementäre Sequenzen, welche auf durch Markierung codierten Trägern immobilisiert sind,
wobei die Markierung der Träger über Fluoreszenzfarbstoffe und/oder
über absorbierende Farbstoffe und/oder über Chemiluminiszenz und/oder
über Transponder und/oder
über Nuklide welche über Elektronenspinresonanz und/oder über Kernspinnresonanz oder radioaktiven Zerfall nachweisbar sind
oder über chemische Markierungen, welche sich massenspektroskopisch nachweisen lassen erfolgt;
b) Hybridisierung von Sonden an die in Schritt a) hybridisierten Nukleinsäurefragmente;
c) sequentielle Identifikation der codierten Träger und Analyse der an diese gebundenen Sonden in einem Massenspektrometer;
d) Zuordnung der erhaltenen Masseninformation zu den Sequenzen der verwendeten Sonden und Assoziierung der Analysenergebnisse mit der Codierung der Träger; e) Abgleich der so erhaltenen Informationen mit einer Datenbank.

2. Verfahren nach Anspruch 1, **dadurch gekennzeichnet, daß** die in Schritt a) hybridisierten Nukleinsäurefragmente DNA sind.

3. Verfahren nach Anspruch 1, **dadurch gekennzeichnet, daß** die in Schritt a) hybridisierten Nukleinsäürefragmente RNA sind.

4. Verfahren nach Anspruch 1, **dadurch gekennzeichnet, daß** die in Schritt a) hybridisierten Nukleinsäurefragmente durch die Polymerase Kettenreaktion erhältlich sind.

5. Verfahren nach einem der vorangehenden Ansprüche, **dadurch gekennzeichnet, daß** die in Schritt a) hybridisierten Nukleinsäurefragmente durch Restriktionsverdau erhältlich sind.

6. Verfahren nach einem der vorangehenden Ansprüche, **dadurch gekennzeichnet, daß** die in Schritt a) hybridisierten Nukleinsäurefragmente durch Behandlung mit einer reversen Transkriptase und anschließender Polymerase Kettenreaktion erhältlich sind.

7. Verfahren nach einem der vorangehenden Ansprüche, **dadurch gekennzeichnet, daß** die in Schritt b) verwendeten Sonden selbst Nukleinsäuren sind.

8. Verfahren nach einem der vorangehenden Ansprüche, **dadurch gekennzeichnet, daß** die in Schritt b) verwendeten Sonden PNA, Alkylphosphonat-DNA, Phosphorothioat-DNA oder alkylierte Phosphorothioat-DNA sind.

9. Verfahren nach einem der vorangehenden Ansprüche, **dadurch gekennzeichnet, daß** die in Schritt b) verwendeten Sonden entweder eine einzelne positive oder negative Nettoladung tragen.

10. Verfahren nach einem der vorangehenden Ansprüche **dadurch gekennzeichnet, daß** die in Schritt b) verwendeten Sonden chemische Gruppen tragen, welche deren Molekülmasse verändern.

11. Verfahren nach einem der vorangehenden Ansprüche, **dadurch gekennzeichnet, daß** die in Schritt b) verwendeten Sonden abspaltbare Gruppen enthalten, welche über deren Masse identifizierbar sind.

12. Verfahren nach einem der vorangehenden Ansprüche, **dadurch gekennzeichnet, daß** jede der in Schritt b) verwendeten Sondensequenzen über deren Sondenmasse identifizierbar ist.

13. Verfahren nach einem der vorangehenden Ansprüche, **dadurch gekennzeichnet, daß** die in Schritt b) verwendeten Sonden durch kombinatorische Synthese erhältlich sind.

14. Verfahren nach einem der Ansprüche 4 oder 6, **dadurch gekennzeichnet, daß** man an den Trägern zu den Primern aus der Amplifikation komplementäre Sequenzen bindet.

15. Verfahren nach Anspruch 1, **dadurch gekennzeichnet, daß** man die Schritte a) und b) gleichzeitig ausführt.

16. Verfahren nach einem der vorangehenden Ansprüche, **dadurch gekennzeichnet, daß** die in der Amplifikation verwendeten Primer Fluoreszenzmarkierungen tragen, die eine Vorauswahl der Träger vor der Analyse zulassen.

17. Verfahren nach einem der vorangehenden Ansprüche, **dadurch gekennzeichnet, daß** man die Träger vor der Durchführung des Schrittes c) aufreiht, identifiziert und nacheinander einer Analyse zuführt.

18. Verfahren nach einem der vorangehenden Ansprüche, **dadurch gekennzeichnet, daß** man die Träger vor der Durchführung des Schrittes c) derart auf eine Oberfläche verteilt, daß an vorbestimmten Orten jeweils nur ein Träger positioniert ist.

19. Verfahren nach einem der vorangehenden Ansprüche, **dadurch gekennzeichnet, daß** man die Sonden vor, bei oder nach der Einführung ins Massenspektrometer vom Träger löst.

20. Verfahren nach einem der vorangehenden Ansprüche, **dadurch gekennzeichnet, daß** man zur Desorption eine Matrix zugibt.

21. Verfahren nach einem der vorangehenden Ansprüche, **dadurch gekennzeichnet, daß** man die Analyse mittels MALDI-Massenspektrometrie durchführt.

22. Verfahren nach einem der Ansprüche 1 bis 15, **dadurch gekennzeichnet, daß** man die Analyse mittels ESI-Massenspektrometrie durchführt.

23. Verfahren nach einem der vorangehenden Ansprüche **dadurch gekennzeichnet, daß** man eine Ionenfalle in der massenspekrometrischen Analyse einsetzt.

24. Verfahren nach einem der vorangehenden Ansprüche, **dadurch gekennzeichnet, daß** die Identifikation des Trägers und die Analyse der hybridisierten Sonden in einem Verfahrensschritt erfolgen.

25. Verfahren nach einem der vorangehenden Ansprüche, **dadurch gekennzeichnet, daß** man die in Schritt a) eingesetzte DNA zuvor mit Sulfit oder Disulfit oder einer anderen Chemikalie derart behandelt, daß alle nicht an der 5-Position der Base methylierten Cytosinbasen derart verändert werden, daß eine dem Basenpaarungsverhalten nach unterschiedliche Base entsteht, während die an der 5-Position methylierten Cytosine unverändert bleiben.

26. Kit, zur Durchführung eines Verfahrens gemäß Anspruch 1, enthaltend
einen Träger mit gebundenen DNA-Sequenzen, der durch Markierung gemäß Anspruch 1 codiert ist und
über ihre Sondenmassen identifizierbare Nukleinsäuresonden.

## Claims

1. A method for the analysis of nucleic acid sequences, **characterized in that** the following steps are conducted:
a) hybridization of nucleic acid fragments to complementary sequences, which are immobilized on supports coded by labels,
wherein the labelling of the supports is detected by means of fluorescent dyes, and/or
by means of absorbing dyes and/or by means of chemiluminescence, and/or
by means of transponders, and/or
by means of nuclides, which are detectable by means of electron spin resonance or by means of radioactive decomposition,
or by means of chemical labels, which can be detected mass-spectroscopically;
b) hybridization of probes to the nucleic acid fragments hybridized in step a);
c) sequential identification of the coded supports and analysis of the probes bound to the latter in a mass spectrometer;
d) assignment of the obtained mass information to the sequences of the probes used and associating of the analysis data with the coding of the supports;
e) matching of the information obtained with a database.

2. The method according to claim 1, **characterized in that** the nucleic acid fragments hybridized in step a) are DNA.

3. The method according to claim 1, **characterized in that** the nucleic acid fragments hybridized in step a) are RNA.

4. The method according to claim 1, **characterized in that** the nucleic acid fragments hybridized in step a) can be obtained by polymerase chain reaction.

5. The method according to one of the preceding claims, **characterized in that** the nucleic acid fragments hybridized in step a) can be obtained by restriction digestion.

6. The method according to one of the preceding claims, **characterized in that** the nucleic acid fragments hybridized in step a) can be obtained by treatment with a reverse transcriptase and subsequent polymerase chain reaction.

7. The method according to one of the preceding claims, **characterized in that** the probes used in step b) are themselves nucleic acids.

8. The method according to one of the preceding claims, **characterized in that** the probes used in step b) are PNA, alkyl phosphonate DNA, phosphorothioate DNA or alkylated phosphorothioate DNA.

9. The method according to one of the preceding claims, **characterized in that** the probes used in step b) bear either a single positive or negative net charge.

10. The method according to one of the preceding claims, **characterized in that** the probes used in step b) bear chemical groups, which modify their molecular mass.

11. The method according to one of the preceding claims, **characterized in that** the probes used in step b) contain cleavable groups, which can be identified by means of their mass.

12. The method according to one of the preceding claims, **characterized in that** each of the probe sequences used in step b) can be identified by means of its probe mass.

13. The method according to one of the preceding claims, **characterized in that** the probes used in step b) can be obtained by combinatorial synthesis.

14. The method according to one of the claims 4 or 6, **characterized in that** sequences complementary to the primers from the amplification are bound to the supports.

15. The method according to claim 1, **characterized in that** steps a) and b) are conducted simultaneously.

16. The method according to one of the preceding claims, **characterized in that** the primers used in the amplification bear fluorescent labels, which permit a preselection of supports prior to the analysis.

17. The method according to one of the preceding claims, **characterized in that** prior to conducting step c) the supports are lined up, identified, and introduced one after the other to an analysis.

18. The method according to one of the preceding claims, **characterized in that** before conducting step c), the supports are distributed on a surface in such a way that only one support is positioned each time at predetermined sites.

19. The method according to one of the preceding claims, **characterized in that** the probes are removed from the support before, during or after introduction into the mass spectrometer.

20. The method according to one of the preceding claims, **characterized in that** a matrix is added for the desorption.

21. The method according to one of the preceding claims, **characterized in that** the analysis is conducted by means of MALDI mass spectrometry.

22. The method according to one of claims 1 to 15, **characterized in that** the analysis is conducted by means of ESI mass spectrometry.

23. The method according to one of the preceding claims, **characterized in that** an ion trap is utilized in the mass-spectrometric analysis.

24. The method according to one of the preceding claims, **characterized in that** the identification of the support and the analysis of the hybridized probes is conducted in one method step.

25. The method according to one of the preceding claims, **characterized in that** the DNA utilized in step a) is treated beforehand with sulfite or disulfite or another chemical in such a way that all cytosine bases that are unmethylated at the 5-position of the base are modified in such a way that a base is formed that is different in its base-pairing behavior, while the cytosines methylated at the 5-position remain unchanged.

26. A kit for conducting a method according to claim 1, containing
a support with bound DNA sequences, which is coded by labels according to claim 1, and
nucleic acid probes identifiable by the mass of the probes thereof.

## Revendications

1. Procédé d'analyse de séquences d'acides nucléiques, **caractérisé en ce qu'**on exécute les étapes suivantes :
a) hybridation de fragments d'acides nucléiques à des séquences complémentaires qui sont immobilisées sur des supports codés par marquage, le marquage des support étant réalisé par l'intermédiaire de colorants fluorescents et/ou de colorants absorbants et/ou d'une chimioluminescence et/ou de transpondeurs et/ou de nucléides, qui peuvent être détectés par résonance paramagnétique des électrons et/ou par résonance magnétique nucléaire ou décomposition radioactive, ou par l'intermédiaire de marquages chimiques pouvant être détectés par spectrométrie de masse ;
b) hybridation de sondes aux fragments d'acide nucléique hybridés dans l'étape a) ;
c) identification séquentielle des supports codés, et analyse des sondes liées à ces derniers dans un spectromètre de masse ;
d) affectation des informations de masse obtenues aux séquences des sondes utilisées, et association des résultats de l'analyse au codage des supports ;
e) comparaison des informations ainsi obtenues avec une banque de données.

2. Procédé selon la revendication 1, **caractérisé en ce que** les fragments d'acides nucléiques hybridés dans l'étape a) sont des ADN.

3. Procédé selon la revendication 1, **caractérisé en ce que** les fragments d'acides nucléiques hybridés dans l'étape a) sont des ARN.

4. Procédé selon la revendication 1, **caractérisé en ce que** les fragments d'acides nucléiques hybridés dans l'étape a) peuvent être obtenus par une réaction en chaîne par polymérase.

5. Procédé selon l'une des revendications précédentes, **caractérisé en ce que** les fragments d'acides nucléiques hybridés dans l'étape a) peuvent être obtenus par digestion par des enzymes de restriction.

6. Procédé selon l'une des revendications précédentes, **caractérisé en ce que** les fragments d'acides nucléiques hybridés dans l'étape a) peuvent être obtenus par traitement avec une transcriptase inverse, suivi d'une réaction en chaîne par polymérase.

7. Procédé selon l'une des revendications précédentes, **caractérisé en ce que** les sondes utilisées dans l'étape b) sont elles-mêmes des acides nucléiques.

8. Procédé selon l'une des revendications précédentes, **caractérisé en ce que** les sondes utilisées dans l'étape b) sont des sondes PNA, alkylphosphonate-ADN, phosphorothioate-ADN, ou phosphorothioate-alkylé ADN.

9. Procédé selon l'une des revendications précédentes, **caractérisé en ce que** les sondes utilisées dans l'étape b) portent une charge nette individuelle positive ou négative.

10. Procédé selon l'une des revendications précédentes, **caractérisé en ce que** les sondes utilisées dans l'étape b) portent des groupes chimiques qui modifient leur masse moléculaire.

11. Procédé selon l'une des revendications précédentes, **caractérisé en ce que** les sondes utilisées dans l'étape b) contiennent des groupes dissociables pouvant être identifiés par leur masse.

12. Procédé selon l'une des revendications précédentes, **caractérisé en ce que** chacune des séquences des sondes utilisées dans l'étape b) peut être identifiée par la masse de sa sonde.

13. Procédé selon l'une des revendications précédentes, **caractérisé en ce que** les sondes utilisées dans l'étape b) peuvent être obtenues par synthèse combinatoire.

14. Procédé selon l'une des revendications 4 ou 6, **caractérisé en ce qu'**on lie au support des séquences complémentaires des amorces provenant de l'amplification.

15. Procédé selon la revendication 1, **caractérisé en ce qu'**on exécute simultanément les étapes a) et b).

16. Procédé selon l'une des revendications précédentes, **caractérisé en ce que** les amorces utilisées lors de l'amplification portent des marquages fluorescents, qui permettent une présélection des supports avant l'analyse.

17. Procédé selon l'une des revendications précédentes, **caractérisé en ce qu'**on place en série les supports avant la mise en oeuvre de l'étape c), on les identifie et on les soumet l'un après l'autre à une analyse.

18. Procédé selon l'une des revendications précédentes, **caractérisé en ce qu'**on répartit les supports, avant mise en oeuvre de l'étape c), sur une surface de telle sorte qu'un seul support soit positionné sur chacun de plusieurs sites prédéfinis.

19. Procédé selon l'une des revendications précédentes, **caractérisé en ce qu'**on sépare du support les sondes avant, pendant ou après introduction dans le spectromètre de masse.

20. Procédé selon l'une des revendications précédentes, **caractérisé en ce que**, pour la désorption, on ajoute une matrice.

21. Procédé selon l'une des revendications précédentes, **caractérisé en ce qu'**on met en oeuvre l'analyse par une spectrométrie de masse MALDI.

22. Procédé selon l'une des revendications 1 à 15, **caractérisé en ce qu'**on met en oeuvre l'analyse par une spectrométrie de masse ESI.

23. Procédé selon l'une des revendications précédentes, **caractérisé en ce qu'**on utilise un piège ionique lors de l'analyse par spectrométrie de masse.

24. Procédé selon l'une des revendications précédentes, **caractérisé en ce que** l'identification du support et l'analyse des sondes hybridées s'effectuent en une étape.

25. Procédé selon l'une des revendications précédentes, **caractérisé en ce qu'**on traite l'ADN utilisé dans l'étape a) d'abord avec un sulfite ou un disulfite ou un autre composé chimique, de telle sorte que les bases cytosines qui ne sont pas méthylées sur la position 5 de la base soient modifiées de façon qu'il se crée une base, différentes pour ce qui est de son comportement d'appariement des bases, tandis que les cytosines méthylées sur la position 5 restent inchangées.

26. Kit destiné à mettre en oeuvre un procédé selon la revendication 1, contenant un support comportant des séquences d'ADN liées, qui est codé par marquage selon la revendication 1, et des sondes d'acides nucléiques identifiables par leur masse de sonde.
